(19) Europäisches Patentamt
European Patent Office
Office européen des brevets

(11) Publication number: 0 226 635 B1

(12) **EUROPEAN PATENT SPECIFICATION**

(45) Date of publication of the patent specification: **06.09.89**

(51) Int. Cl.⁴: **C 07 C 51/41**, C 08 F 22/06, C 08 F 30/02, C 08 F 28/02, C 08 G 63/62

(21) Application number: **86904599.7**

(22) Date of filing: **02.07.86**

(86) International application number: **PCT/US 86/01388**

(87) International publication number: **WO 87/00520 (29.01.87** Gazette **87/3)**

# (54) METHOD FOR NEUTRALIZATION OF ORGANOPHILIC ACIDIC COMPOUNDS.

(30) Priority: **26.07.85 US 759198**

(43) Date of publication of application: **01.07.87 Bulletin 87/27**

(45) Publication of the grant of the patent: **06.09.89 Bulletin 89/36**

(84) Designated Contracting States: **DE FR GB IT NL**

(56) References cited:
**GB-A- 684 155**
**GB-A- 1 136 884**
**US-A- 3 225 075**

(73) Proprietor: **GENERAL ELECTRIC COMPANY, 1 River Road, Schenectady New York 12305 (US)**

(72) Inventor: **BROWN, Sterling, Bruce, 3 Rosa Road, Schenectady, NY 12308 (US)**
Inventor: **YATES, John, Bennie, III, 38 Chrisken Drive, Gelmont, New York 12077 (US)**

(74) Representative: **Catherine, Alain, General Electric France Service de Propriété Industrielle 18 Rue Horace Vernet B.P. 76, F-92134 Issy-les-Moulineaux Cedex (FR)**
Representative: **Sieb, Rolf, Dr., General Electric - Deutschland Patentabteilung Praunheimer Landstrasse 50, D-6000 Frankfurt/Main (DE)**

**EP 0 226 635 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

**Description**

This invention relates to the neutralization of organophilic acidic compounds and their derivatives, and more particularly to a method for such neutralization in a water-free system of low polarity.

It is frequently necessary to prepare metal salts or organophilic acidic compounds, as illustrated by sulfonic and carboxylic acids, or of functional derivatives thereof. For the most part, neutralization of such compounds has been conducted in an aqueous or alcoholic solution of a metal hydroxide or salt such as sodium hydroxide, potassium hydroxide, zinc acetate or barium acetate. However, there are instances in which the use of an aqueous or highly polar system of this type is undesirable. Certain highly organophilic compounds such as sulfonated elastomers, for example, are much more soluble in non-polar liquids than in polar ones such as alcohols. Moreover, sulfonated polycarbonates and certain other acidic organic compounds are degraded by water and to some extent by polar solvents such as alcohols, making impossible the use of such solvents in substantial amounts for their neutralization.

A principal object of the present invention, therefore, is to provide a method for neutralization of organophilic acidic compounds and derivatives thereof which does not require the use of highly polar solvent systems such as water or alcohols.

A further object is to provide such a method which is adaptable to the preparation of salts of a large number of metals and which utilizes metal compounds which are readily available or which may be easily obtained.

Other objects will in part be obvious and will in part appear hereinafter.

In its broadest sense, the present invention is a method for preparing a metal salt of an organophilic acidic compound or functional derivative thereof which comprises intimately contacting said compound under substantially non-polar conditions with a metal salt of at least one polyketone containing at least two carbonyl groups separated by a single CH moiety.

The term «organophilic» when used herein denotes a higher degree of compatibility with and solubility in non-polar organic liquids than water and other polar liquids. As a general rule, the organophilic compounds contain large organic moieties and a relatively small proportion of polar (acidic) groups.

An extremely broad scope of organophilic acidic compounds is capable of neutralization by the method of this invention. Examples of such compounds will be readily apparent to those skilled in the art. They include monomeric compounds such as fatty acids, alkanesulfonic acids and alkylbenzenesulfonic acids, especially those containing at least about 12 carbon atoms. They also include ionomeric polymers containing organic substituents such as carboxylic, sulfonic and phosphorus acid groups, generally present in the amount of about 1-5% of the total number of structural units in the polymer. Functional derivatives of such acidic compounds, such as esters, amides and anhydrides, may also be converted to salts according to the invention. Illustrative polymers are graft copolymers of olefins and/or dienes with maleic anhydride, olefin and/or diene polymers containing phosphonic acid or phosphonic acid ester groups, oxidized olefin polymers, sulfonated elastomers, polysiloxane sulfonates, sulfonated polycarbonates, and sulfonated polyimides of the type disclosed in copending, commonly owned applications Serial No. 647 596, filed September 6, 1984, and Serial No. 682 202, filed December 17, 1984.

The metals whose salts may be prepared by the method of this invention include virtually all salt-forming metals. Non-limiting examples are lithium, sodium, potassium, cesium, beryllium, magnesium, calcium, strontium, barium, yttrium, titanium, vanadium, chromium, manganese, iron, cobalt, nickel, ruthenium, rhodium, palladium, iridium, platinum, copper, silver, zinc, cadmium, aluminum, gallium, indium, thallium, lead, lanthanum, cerium, samarium, gadolinium, terbium, erbium and thulium. The preparation of zinc salts is particularly facile. In addition to the simple metal salts, metal oxy salts may also be produced by this method.

According to the invention, the organophilic acidic compound or derivative thereof is treated with a metal salt of at least one polyketone in which at least two carbonyl groups are separated by a single CH moiety. Illustrative polyketones are those having the formula.

$$R^1-\overset{\overset{O}{\|}}{C}-\underset{\underset{X}{|}}{CH}-\overset{\overset{O}{\|}}{C}-R^2,$$

wherein each of $R^1$ and $R^2$ is an alkyl, fluoroalkyl, aromatic or substantially neutral heterocyclic radical and X is hydrogen, chloro, alkyl or acyl, or $R^2$ and X taken together are alkylene, cycloalkylene or a keto-, fluoro- or aryl-substituted derivative thereof.

The following are examples of polyketones which may be used according to this invention:

2,4-pentanedione
2,2-dimethyl-3,5-heptanedione
3-chloro-2,4-pentanedione
1,1,1-trifluoro-2,4-pentanedione
2,2-dimethyl-6,6,7,7,8,8,8-heptafluoro-3,5-octanedione
3-acetyl-2,4-pentanedione
1-phenyl-1,3-butanedione
1,3-diphenyl-1,3-propanedione
1-phenyl-4,4,4-trifluoro-1,3-butanedione
1-(3-thienyl)-4,4,4-trifluoro-1,3-butanedione
1,7,7-trimethyl-3-trifluoroacetyl(2.2.1)bicycloheptane-2-one.

The presence of a CH moiety linking two carbonyl groups is essential, since the hydrogen atom therein is the source of the proton which is replaced by the metal ion. Those skilled in the art are aware that the resulting anion is a hybrid in which the negative charge is delocalized over the carbon atom of the CH group and the adjacent carbonyl oxygen atoms. The simplest such polyketone is 2,4-pentanedione (acetylacetone), in which $R^1$ and $R^2$ are each methyl and X is hydrogen, which is preferred for use in the invention because of its relative availability and low cost.

According to the invention, the organophilic compound and the polyketone metal salt (or a hydrate thereof) are intimately contacted under substantially non-polar conditions. In general, such contact involves the liquid phase, at least in part. Thus, the reagents may be meltblended or may be dissolved in at least one substantially inert, relatively non-polar organic liquid such as methylene chloride, chloroform, 1,2-dichloroethane, heptane, petroleum naphtha, benzene, toluene, xylene, chlorobenzene, o-dichlorobenzene or dioxane. The identity of the liquid, if any, is not critical and the choice may be made according to the solubilities of the reagents therein.

The proportions of reagents are also not critical and may be chosen according to the degree of salt formation desired. For stoichiometric conversion of the acidic compound to the metal salt, an amount of the metal polyketone salt from stoichiometric to about 50% excess is typically employed.

Any desired concentration of the reagents in the solvent (if used) is useful. Most often, an approximately 1-30% (by weight) solution of the organophilic compound will be prepared and the polyketone salt will be combined therewith in the desired proportion. Reaction temperatures in the range of about 0-200°C are usually employed, although lower or higher temperatures may also be suitable.

If desired, the salts obtained by the method of this invention may be isolated by conventional means, such as solvent evaporation or precipitation by adding a non-solvent for the salt. It is also within the scope of the invention to employ the salt in solution rather than isolating it.

The method of this invention is illustrated by the following examples.

*Example 1*

A solution in 2250 ml. of toluene of 100 grams of a polymer containing 0.5% maleic anhydride grafted on polyethylene was heated under reflux, with stirring, and a slurry of 1.5 grams of zinc 2,4-pentanedionate hydrate in 100 ml. of toluene was added incrementally. A gel was formed which was added to 2 volumes of methanol with vigorous stirring. The opaque white precipitate was allowed to settle and the liquid was decanted. The solid was stirred with 2 liters of methanol for 30 minutes, collected by filtration, washed with methanol and dried. The product was the desired zinc salt.

*Example 2*

Following substantially the procedure of Example 1, 1.5 grams of zinc 2,4-pentanedionate hydrate was added incrementally to a solution in 2500 ml. of toluene of 94.6 grams of a phosphonated polyethylene containing 0.18% phosphorus, prepared by the reaction of polyethylene with phosphorus trichloride and oxygen followed by hydrolysis. The desired zinc salt was obtained.

*Example 3*

A reaction product of diethyl vinylphosphonate and an ethylene-propylene rubber, containing 0.51% phosphorus, was dissolved in a mixture of 1000 ml. of toluene and 50 ml. of dioxane and was converted to the free phosphonic acid by treatment with hydrogen chloride gas at 75°C. The solution was sparged with nitrogen to remove hydrogen chloride, and 1.02 grams of solind zinc 2,4-pentanedionate hydrate was added, with stirring. Within two minutes, a gel had formed which was worked up as in Example 1 to yield the desired zinc salt.

*Example 4*

Anhydrous zinc 2,4-pentanedionate, 62 mg., was added with stirring to a solution of 5 grams of the graft copolymer of Example 1 in 150 ml. of toluene. Upon workup as in Example 1, the desired zinc salt was obtained.

*Examples 5-7*

Following substantially the procedure of Example 1, zinc 2,4-pentanedionate hydrate was reacted with the following polymers in the indicated solvents:

*Example 5* A phosphonated ethylene-propylene rubber similar to the phosphonated polymer of Example 2; o-dichlorobenzene.

*Example 6* A graft copolymer of maleic anhydride on an EPDM rubber; o-dichlorobenzene.

*Example 7* An oxidized polyethylene with an acid number of 28; toluene.

*Example 8*

A 2:1 sulfur trioxide-triethyl phosphate complex was prepared by adding 3.38 grams (42.2 mmol.) of sulfur trioxide dropwise under nitrogen at 0°C, with stirring, to a solution of 3.85 grams (21.1 mmol.) of triethyl phosphate in 300 ml. of 1,2-dichloroethane. Stirring was continued for 15 minutes, after which the complex solution was added to a solution in 3.5 liters of 1,2-dichloroethane of 125 grams (211 mmol. based on structural units) of a polyetherimide having a number average molecular weight (as determined by gel permeation chromatography) of about 23,000, prepared by the reaction of substantially equimolar amounts of 2,2-bis-[4-(3,4-dicarboxyphenoxy)-phenyl]propane dianhydride and m-phenylenediamine. Addition was effected rapidly at room temperature, with stirring which was continued for 30 minutes. 2-Propanol, 150 ml., was then added and the turbid solution was stirred until clear.

Zinc 2,4-pentanedionate hydrate, 148.2 grams, was added and the solution was stirred for one hour. It was then poured into 10 liters of methanol, whereupon the zinc salt of the sulfonated polymer precipitated. It was extracted with water for 12 hours and dried in a vacuum oven at 80°C.

*Example 9*

Zinc 2,4-pentanedionate hydrate, 23 mg., was mixed in a slurry with one gram of a graft copolymer of maleic anhydride on polyethylene similar to that of Example 1, but containing 1.5% maleic anhydride, together with a small amount of acetone. The mixture was pressed on a Carver press between polytetrafluoroethylene-coated metal sheets at 140°C and 5000 psi. for one minute, cut into small pieces and repressed at 140°C and 20,000 psi. for one minute. The pressing operation was repeated two more times to yield the desired zinc salt.

*Example 10*

Zinc 2,4-pentanedionate hydrate, 500 mg., was blended at 170°C for 10 minutes with 25 grams of the polyethylene-maleic anhydride graft polymer of Example 1. The product was the desired zinc salt.

*Example 11*

One gram of zinc 2,4-pentanedionate hydrate was blended at 170°C for 10 minutes with 30 grams of graft copolymer of maleic anhydride on an EPDM (ethylene-propylene-diene terpolymer), containing 1.5% maleic anhydride units. The desired zinc salt was obtained.

*Example 12*

To a solution in 2.25 liters of 1,2-dichloroethane of 102 grams of a sulfonated bisphenol A polycarbonate having a weight average molecular weight of about 71,000 (as determined by gel permeation chromatography) and containing about 1.2% sulfur is added, with stirring, 20 ml. of 2-propanol and 15.4 grams of potassium 2,4-pentanedionate. Stirring is continued for one hour, after which the mixture is concentrated to about 1 liter by vacuum evaporation and poured into 8 liters of methanol. The desired potassium salt of the sulfonated polycarbonate is collected by filtration and dried in a vacuum oven. It contains about 1.4% potassium.

## Claims

1. A method for preparing a metal salt of an organophilic acidic compound or functional derivative thereof which comprises intimately contacting said compound under substantially non-polar conditions with a metal salt of at least one polyketone containing at least two carbonyl groups separated by a single CH moiety.

2. A method according to claim 1 wherein the polyketone has the formula

O O
|| ||
$R^1$- C - CH- C -$R^2$,
|
X

wherein each of $R^1$ and $R^2$ is an alkyl, fluoroalkyl, aromatic or substantially neutral heterocyclic radical and X is hydrogen, chloro, alkyl or acyl, or $R^2$ and X taken together are alkylene, cycloalkylene or a keto-, fluoro- or aryl-substituted derivative thereof.

3. A method according to claim 2 in which the polyketone is 2,4-pentanedione.

4. A method according to claim 3 wherein said salt is prepared by melt-blending.

5. A method according to claim 3 wherein said salt is prepared in solution in at least one substantially inert, relatively non-polar organic liquid.

6. A method according to claim 3 wherein the organophilic compound is an ionomeric polymer.

7. A method according to claim 6 wherein the polymer contains carboxylic acid groups.

8. A method according to claim 6 wherein the polymer contains sulfonic acid groups.

9. A method according to claim 6 wherein the polymer contains phosphorus acid or ester groups.

10. A method according to claim 9 wherein the polymer contains phosphonic acid or ester groups.

11. A method according to claim 3 wherein the reaction temperature is in the range of about 0-200°C.

12. A method according to claim 3 wherein the metal is zinc.

## Patentansprüche

1. Verfahren zum Herstellen eines Metallsalzes einer organophilen sauren Verbindung oder eines funktionellen Derivates davon, umfassend das innige Inberührungbringen der genannten Verbindung unter im wesentlichen nicht polaren Bedingungen mit einem Metallsalz mindestens eines Polyketons, das mindestens zwei durch eine einzige CH-Gruppierung getrennte Carbonylgruppen enthält.

2. Verfahren nach Anspruch 1, worin das Polyketon die folgende Formel hat

O O
|| ||
$R^1$- C - CH- C -$R^2$,
|
X

worin jedes $R^1$ und $R^2$ ein Alkyl-, Fluoralkyl-, aromatischer oder im wesentlichen neutraler heterocyclischer Rest ist und X Wasserstoff, Chlor, Alkyl oder Acyl ist oder $R^2$ und X zusammen Alkylen, Cycloalkylen oder ein Keto-, Fluor- oder Aryl-substituiertes Derivate davon sind.

3. Verfahren nach Anspruch 2, bei dem das Polyketon 2,4-Pentandion ist.

4. Verfahren nach Anspruch 3, worin das Salz durch Schmelzvermischen hergestellt wird.

5. Verfahren nach Anspruch 3, worin das Salz in Lösung in mindestens einer im wesentlichen inerten, relativ nicht-polaren organischen Flüssigkeit hergestellt wird.

6. Verfahren nach Anspruch 3, worin die organophile Verbindung ein ionomeres Polymer ist.

7. Verfahren nach Anspruch 6, worin das Polymer Carbonsäuregruppen enthält.

8. Verfahren nach Anspruch 6, worin das Polymer Sulfonsäuregruppen enthält.

9. Verfahren nach Anspruch 6, worin das Polymer Phosphorsäure- oder Estergruppen enthält.

10. Verfahren nach Anspruch 9, worin das Polymer Phosphonsäure- oder Estergruppen enthält.

11. Verfahren nach Anspruch 3, worin die Reaktionstemperatur im Bereich von etwa 0 bis 200°C liegt.

12. Verfahren nach Anspruch 3, worin das Metall Zink ist.

## Revendications

1. Procédé de préparation d'un sel métallique d'un composé acide organophile ou de son dérivé fonctionnel qui comprend la mise en contact intime du composé dans des conditions essentiellement non polaires avec un sel métallique d'au moins une polycétone contenant au moins deux groupes carbonyles séparés par un seul reste CH.

2. Procédé selon la revendication 1, dans lequel la polycétone à pour formule

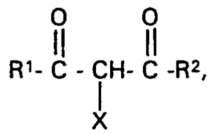

dans laquelle chacun des groupes $R^1$ et $R^2$ représente un radical alkyle, fluoroalkyle, aromatique ou hétérocyclique pratiquement neutre et X représente l'hydrogène, un groupe chloro, alkyle ou acyle ou $R^2$ et X, pris ensemble, représentent un radical alkylène, cycloalkylène ou un de leurs dérivés céto-, fluoro-, ou aryl-substitué.

3. Procédé selon la revendication 2, dans lequel la polycétone est la pentanedione -2,4.

4. Procédé selon la revendication 3, dans lequel on prépare le sel par mélange à l'état fondu.

5. Procédé selon la revendication 3, dans lequel on prépare le sel en solution dans au moins un liquide organique pratiquement inerte, relativement non polaire.

6. Procédé selon la revendication 3, dans lequel le composé organophile est un polymère ionomérique.

7. Procédé selon la revendication 6, dans lequel le polymère contient des groupes acides carboxyliques.

8. Procédé selon la revendication 6, dans lequel le polymère contient des groupes acides sulfoniques.

9. Procédé selon la revendication 6, dans lequel le polymère contient des groupes esters ou acides contenant du phosphore.

10. Procédé selon la revendication 9, dans lequel le polymère contient des groupes esters ou acides phosphoniques.

11. Procédé selon la revendication 3, dans lequel la température de réaction est comprise entre environ 0 et 200°C.

12. Procédé selon la revendication 3, dans lequel le métal est le zinc.